# FASCICULE DE BREVET EUROPEEN

(11) **EP 0 953 345 B1**
(45) Date de publication et mention de la délivrance du brevet: **28.04.2004**
(21) Numéro de dépôt: 99400861.3
(22) Date de dépôt: 08.04.1999
(51) Int. Cl.: A61K 7/48, A61K 7/06, A61K 31/05

(54) **Utilisation d'au moins un hydroxystilbène en tant qu'agent diminuant l'adhésion des micro-organismes**
Verwendung mindestens eines Hydroxystilbene als Mittel zur Verringerung der Adhäsion von Mikroorganismen
Use of at least a hydroxystilbene as an agent for decreasing micro-organisms adhesion

(30) Priorité: 10.04.1998 FR 9804572
(43) Date de publication de la demande: 03.11.1999
(73) Titulaire: L'OREAL, 75008 Paris (FR)
(72) Inventeur: Breton, Lionel, 78000 Versailles (FR); Pineau, Nathalie, Rés. des Jardins du Clain, 86000 Poitiers (FR)
(74) Mandataire: Tezier Herman, Béatrice

(56) Documents cités:
- CHEMICAL ABSTRACTS, vol. 128, no. 13, 30 mars 1998 (1998-03-30) Columbus, Ohio, US; abstract no. 158748, XP002093496 & JP 10 045566 A (YUSHIRO CHEMICAL INDUSTRY CO.) 17 février 1998 (1998-02-17)
- CHEMICAL ABSTRACTS, vol. 87, no. 3, 18 juillet 1977 (1977-07-18) Columbus, Ohio, US; abstract no. 16441, XP002093497 & RATANABANANGKOON K. ET AL.: J. SCI. SOC. THAILAND, vol. 2, no. 4, 1976, pages 202-205, bangkok
- PATENT ABSTRACTS OF JAPAN vol. 098, no. 004, 31 mars 1998 (1998-03-31) & JP 09 328410 A (YUSHIRO CHEM IND CO LTD;MITSUBA BOEKI KK), 22 décembre 1997 (1997-12-22)
- BOMBARDELLI ET AL.: "vitis vinifera l." FITOTERAPIA, vol. 66, no. 4, 1995, pages 291-317, XP002095075 italie

## Description

L'invention se rapporte à l'utilisation d'au moins un hydroxystilbène dans ou pour la préparation d'une composition dermatologique en tant qu'agent diminuant l'adhésion des micro-organismes, particulièrement des bactéries, sur la peau. En particulier l'utilisation des compositions de l'invention sont destinées à favoriser l'élimination des mauvaises odeurs corporelles ou à lutter contre toutes les infections cutanées mettant en jeu des micro-organismes, comme par exemple l'acné et/ou les pellicules.

Il est bien connu que la peau est couverte d'une flore responsable de toute une série de désagréments allant de la simple production d'odeur à des pathologies plus ou moins sévères comme par exemple l'acné et/ou les pellicules.

Les micro-organismes commensaux vivant sur ou dans la peau peuvent faire partie d'une microflore soit résidante (normale) soit transitoire. Les organismes résidants se développent normalement sur ou dans la peau. Leur présence est établie en profils de distribution bien définis. Les micro-organismes présents temporairement sont appelés transitoires. Habituellement, ces organismes ne se fixent pas fermement ; ils sont incapables de se multiplier et meurent normalement après quelques heures.

L'anatomie et la physiologie de la peau varient d'une partie à l'autre du corps et la microflore résidante reflète ces variations.
La plupart des bactéries de la peau sont présentes sur l'épiderme squameux superficiel, colonisant les cellules mortes ou étroitement associées aux glandes sébacées et sudoripares. Les excrétions de ces glandes fournissent de l'eau, des acides aminés, de l'urée, des électrolytes et des acides gras spécifiques servant d'éléments nutritifs principalement pour *Staphylococcus epidermidis* et des corynébactéries aérobies.
Les bactéries Gram-négatives sont généralement présentes dans les régions plus humides.
Les levures *Pityrosporum ovale* et *Pityrosporum orbiculare* sont normalement présentes sur l'épicrâne.
Certains mycètes dermatophytes peuvent coloniser la peau et provoquent des mycoses, comme par exemple le pied d'athlète et la teigne tonsurante.
Certains agents pathogènes présents sur ou dans la peau sont des résidants transitoires colonisant les zones autour des orifices. *Staphylococcus aureus* est le meilleur exemple. Il est présent dans les narines et la région périanale mais survit mal ailleurs. De la même manière, *Clostridium perfringens* colonise habituellement le périnée et les cuisses, particulièrement chez les patients souffrant du diabète.

Théoriquement, l'épiderme n'est pas un environnement favorable pour la colonisation par les micro-organismes. Plusieurs facteurs comme le dessèchement périodique de la peau, le pH légèrement acide de la peau, la concentration élevée en chlorure sodique de la sueur, certaines substances inhibitrices naturelles (bactéricides et/ou bactériostatiques) sont responsables de ce micro-environnement hostile.

L'absence d'humidité induit un état de dormance chez de nombreux résidants de la microflore. Cependant, sur certaines parties du corps (l'épicrâne, les oreilles, les régions axillaires, les régions génito-urinaire et anale, le périnée et les paumes), l'humidité est suffisamment élevée pour permettre l'existence d'une microflore résidante.
Le pH acide (4-6) de la peau, dû aux acides organiques produits par les staphylocoques et aux sécrétions des glandes sébacées et sudoripares, décourage la colonisation par de nombreux micro-organismes.
La sueur contient du chlorure sodique en une concentration qui établit des conditions hyperosmotiques à la surface de la peau et pèse osmotiquement sur la plupart des micro-organismes.
Finalement, certaines substances inhibitrices naturelles aident à contrôler la colonisation, la croissance excessive et l'infection de la surface de la peau par les micro-organismes résidants. Par exemple, les glandes sudoripares excrètent du lysozyme qui lyse *Staphylococcus epidermidis* et d'autres bactéries Gram-positives.
Certaines bactéries Gram-positives (*Propionibacterium acnes)* peuvent changer les lipides sécrétés par les glandes sébacées en acides gras insaturés, comme par exemple l'acide oléique, qui ont une forte activité antimicrobienne sur les bactéries Gram-négatives et les mycètes.

Cependant, dans certaines conditions ce système naturel de défense peut, tout en étant efficace, présenter des désagréments, voire être pris en défaut.

Par exemple certaines substances inhibitrices naturelles (bactéricides et/ou bactériostatiques) dues à la dégradation partielles des lipides complexes sécrétés par les glandes sudoripares sont volatils et peuvent être associés à une forte odeur que l'on a coutume de combattre. Certes de nombreux déodorants contiennent des substances antibactériennes agissant sélectivement sur les bactéries Gram-positives responsables de ces dégradations pour réduire la production d'acides gras insaturés aromatiques et l'odeur corporelle. Mais les déodorants peuvent modifier la microflore, principalement vers les bactéries Gram-négatives, et déclencher en conséquence des infections.
Il demeure donc intéressant de pouvoir disposer dans le cadre du traitement des odeurs corporelles de composés et/ou de compositions efficaces et ne présentant pas d'effets secondaires.

Un autre exemple est *Propionibacterium acnes,* la bactérie le plus fréquemment associée avec les glandes cutanées qui est un bâtonnet Gram-positif, anaérobie et lipophile. Cette bactérie est habituellement inoffensive. Toutefois, on l'a associée à une maladie cutanée, l'acné juvénile. L'acné apparaît ordinairement pendant l'adolescence lorsque le système endocrinien est très actif. L'activité hormonale stimule la surproduction de sébum, un fluide sécrété par les glandes sébacées. Un volume important de sébum s'accumule dans les glandes et fournit un micro-environnement idéal pour *Propionibacterium acnes.* Chez certains individus, cette accumulation déclenche une réponse inflammatoire provoquant une rougeur et un gonflement du canal glandulaire et produisant un comédon, un bouchon de sébum et de kératine dans le canal. Il en résulte des lésions inflammatoires (papules, pustules, nodules), communément appelées "points noirs". *Propionibacterium acnes* semble être l'organisme producteur de lipases qui dégradent les triglycérides du sébum en acide gras libres. Ces dérivés sont particulièrement irritants parce qu'ils peuvent pénétrer dans le derme et promouvoir une inflammation.

Les levures *Pityrosporum ovale* et *Pityrosporum orbiculare* sont communément associées à la formation des pellicules.

Les souches de *Staphylococcus aureus* sont connus comme super antigènes, ce qui favorise l'apparition de réactions d'irritation et de processus inflammatoires.

Bien évidemment, si nécessaire, on sait utiliser depuis longtemps des composés comme les antibiotiques ou certains dérivés naturels ou synthétiques de la vitamine A pour palier aux déficiences de ce système naturel de défense.
Mais, là encore, on sait que l'utilisation de tels composés présente des aspects négatifs non négligeables. L'utilisation abusive d'antibiotiques peut aboutir à l'émergence de micro-organismes résistants sur lesquels elles n'ont plus d'efficacité. Quant aux dérivés de la vitamine A, on sait qu'ils présentent généralement des effets secondaires importants, ce qui rend leur utilisation délicate.
Ainsi, là encore, on a besoin de composés et/ou de compositions efficaces et ne présentant pas d'effets secondaires.

Par ailleurs on sait qu'un des éléments clefs pour qu'un agent pathogène induise une maladie infectieuse est qu'il doit être capable d'adhérer à l'hôte qu'il va coloniser et envahir.

En effet après avoir été transmis à un hôte approprié, l'agent pathogène doit être capable de se fixer et de coloniser les cellules et les tissus de l'hôte. La colonisation dépend de la capacité qu'a le germe pathogène à concurrencer avec succès la microflore normale de l'hôte pour les éléments nutritifs essentiels. Des structures spécialisées, permettant d'entrer en concurrence pour des sites d'attachement en surface, sont également nécessaires à la colonisation.
Les organismes pathogènes, comme beaucoup de non pathogènes, se fixent de manière très spécifique à des tissus particuliers. Les structures d'adhérence sont un des éléments de cette spécificité. Elles sont spécialisées et présentes sur la surface de l'agent pathogène qui se fixent sur des récepteurs spécifiques des cellules hôtes.

On comprend donc qu'une des voies possibles pour traiter les désagréments et/ou les infections cutanées est de lutter contre l'adhésion des micro-organismes aux cellules de la peau et/ou des muqueuses.

De manière surprenante et inattendue, la demanderesse a découvert que les hydroxystilbènes et particulièrement le 3,4'5-trihydroxystilbène encore appelé resvératrol, présentent la particularité de modifier l'attachement des micro-organismes sur les cellules en particulier sur les cellules de la peau et/ou des muqueuses.

Les hydroxystilbènes sont des composés répondant à la formule générale I : 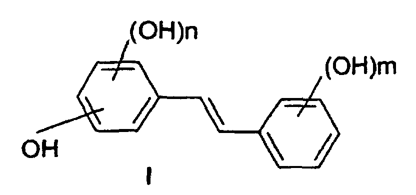 dans laquelle n est un nombre entier compris entre 0 et 4 inclusivement et m est un nombre entier compris entre 0 et 5 inclusivement. Ces composés peuvent être sous une forme Cis ou Trans.
Selon l'invention, le terme hydroxystilbène recouvre aussi bien les composés de formule I que leurs dérivés hydroxyalkylés.

Les hydroxystilbènes sont des composés que l'on retrouve à l'état naturel dans les végétaux de la classe des spermatophytes et particulièrement dans la vigne. On trouve de tels composés comme par exemple le resvératrol dans le raisin et dans le vin.

Dans l'art antérieur les hydroxystilbènes sont utilisés entre autres comme agents dépigmentants (JP87-192040), comme agents vasodilatateurs (EP 96-830517), comme agents antithrombiques (JP 05016413), dans le traitement de diverses affections cardio-vasculaires (CA 2187990), comme agents inhibiteurs de mutagenèse et de carcinogenèse (JP 06024967), ou encore décrits comme antioxydants.

JP 10 045 566 décrit des compositions nettoyantes, en particulier sous forme de savon comprenant des extraits de Yucca associant 3 familles de composés : des saponines, des flavones et du resvératrol. Ces compositions ont un effet moussant, hydratant et antimicrobien, et sont utilisées pour prévenir la peau rugueuse. Il n'est fait aucune mention d'un effet 'anti-adhésion' vis à vis des bactéries et/ou des levures.

Parmi ces composés le resvératrol (ou 3, 5, 4'-trihydroxystilbène) est particulièrement étudié pour les activités ci-dessus décrites principalement parce qu'il s'agit d'un composé naturel qui se retrouve dans la peau des grains de raisin et dans le vin. A cet égard on peut consulter la revue de Soleas et collaborateurs (Clinical Biochemistry, vol. 30, N°2, pp. 91-113, 1997) qui résume parfaitement l'état des connaissances concernant ce composé et les hydroxystilbènes.

Mais, la capacité des hydroxystilbènes à modifier l'attachement des micro-organismes sur les cellules en particulier sur les cellules de la peau et/ou des muqueuses n'a jamais été décrite à ce jour.

L'invention a donc pour objet l'utilisation, en tant que principe actif, dans ou pour la préparation d'une composition dermatologique d'une quantité efficace d'au moins un hydroxystilbène, cet hydroxystilbène ou la composition étant destinés à modifier l'adhésion des micro-organismes sur la peau et/ou les muqueuses.

Parmi les hydroxystilbènes, on peut citer les mono, di, tri, tétra, penta, hexa, hepta, octo, nonahydroxystilbènes, ou encore leurs dérivés hydroxyalkylés.

Par principe actif, on entend toute molécule ou composition susceptible de modifier ou de moduler le fonctionnement d'au moins un système biologique donné.

Par "modifier l'adhésion des micro-organismes" il faut entendre la capacité à prévenir, totalement ou partiellement, l'adhésion des micro-organismes, ou la capacité à titre curatif à faciliter le détachement des micro-organismes.

Ainsi, l'invention a pour objet l'utilisation, en tant que principe actif, dans ou pour la préparation d'une composition dermatologique d'une quantité efficace d'au moins un hydroxystilbène, cet hydroxystilbène ou la composition étant destinés à prévenir, totalement ou partiellement, l'adhésion des micro-organismes sur la peau et/ou les muqueuses.

De même l'invention a pour objet l'utilisation, en tant que principe actif, dans ou pour la préparation d'une composition dermatologique d'une quantité efficace d'au moins un hydroxystilbène, cet hydroxystilbène ou la composition étant destinés à faciliter le détachement des micro-organismes de la peau et/ou les muqueuses.

Particulièrement l'hydroxystilbène ou la composition le contenant est utilisé selon l'invention en application topique sur la peau et/ou les muqueuses.

On a vu précédemment que l'adhésion de micro-organismes à la peau et/ou aux muqueuses a des conséquences qui vont du simple désagrément (l'odeur) aux maladies plus ou moins graves.

Un des aspects de l'invention est donc de proposer l'utilisation d'au moins un hydroxystilbène en tant qu'actif dans ou pour la préparation d'une composition dermatologique cet hydroxystilbène ou la composition étant destinés aux soins d'hygiène corporelle, y compris les soins intimes.

Par soins d'hygiène corporelle on entend toutes substances ou préparations destinées à être mises en contact avec les diverses parties superficielles du corps humain et/ou avec les dents et/ou les muqueuses en vue de les nettoyer, de les protéger, de les maintenir en bon état, d'en modifier l'aspect, de les parfumer ou d'en corriger l'odeur.
Particulièrement, l'invention a pour objet l'utilisation d'au moins un hydroxystilbène en tant qu'actif dans ou pour la préparation d'une composition dermatologique cet hydroxystilbène ou la composition étant destinés à diminuer les mauvaises odeurs corporelles.

On a vu précédemment que la flore bactérienne de la surface de la peau est responsable d'un grand nombre de désordres.

Ainsi, l'invention a également pour objet l'utilisation d'au moins un hydroxystilbène en tant qu'actif dans ou pour la préparation d'une composition dermatologique cet hydroxystilbène ou la composition étant destinés à lutter contre les mycoses, l'acné, particulièrement l'acné juvénile et/ou les pellicules.

Particulièrement, l'invention a pour objet l'utilisation d'au moins un hydroxystilbène en tant qu'actif dans ou pour la préparation d'une composition dermatologique cet hydroxystilbène ou la composition étant destinés à lutter contre l'acné et/ou les pellicules.

Selon l'invention les hydroxystilbènes peuvent être utilisés seuls ou en mélanges de toute nature et peuvent être d'origine naturelle ou synthétique.

Les hydroxystilbènes utilisables selon l'invention sont choisis parmi :
le 4'-hydroxystilbène,
le 2',4'-dihydroxystilbène,
le 3',4'-dihydroxystilbène,
le 4,4'-dihydroxystilbène,
le 2',4',4-trihydroxystilbène,
le 3',4',4-trihydroxystilbène,
le 2,4,4'-trihydroxystilbène,
le 3,4,4'-trihydroxystilbène,
le 3,4',5-trihydroxystilbène,
le 2',3,4-trihydroxystilbène,
le 2,3',4-trihydroxystilbène,
le 2',2,4'-trihydroxystilbène,
le 2,4,4',5-tétrahydroxystilbène,
le 2',3,4',5-tétrahydroxystilbène,
le 2,2',4,4'-tétrahydroxystilbène,
le 3,3',4',5-tétrahydroxystilbène,
le 2,3',4,4'-tétrahydroxystilbène,
le 3,3',4,4'-tétrahydroxystilbène,
le 3,3', 4',5, 5'-pentahydroxystilbène,
le 2,2',4,4',6-pentahydroxystilbène,
le 2, 3',4,4',6-pentahydroxystilbène,
le 2,2',4,4',6,6'-hexahydroxystilbène.

Préférentiellement, on utilise selon l'invention du 3,4',5-trihydroxystilbène (ou resvératrol).

La quantité d'hydroxystilbène utilisable selon l'invention dépend bien évidemment de l'effet recherché et doit être en une quantité efficace pour empêcher partiellement, voire totalement, l'adhésion des micro-organismes ou pour faciliter le détachement des micro-organismes.

A titre d'exemple la quantité d'hydroxystilbène utilisable selon l'invention peut aller par exemple de 0,001 % à 10 % et de préférence de 0,005 % à 5 % du poids total de la composition.

L'invention a également pour objet un procédé cosmétique pour traiter les désordres liés à l'adhésion de micro-organismes consistant à appliquer sur la peau une composition cosmétique comprenant au moins un hydroxystilbène dans un milieu cosmétiquement acceptable.

Par milieu cosmétiquement acceptable, on entend compatible avec la peau, le cuir chevelu les muqueuses, les ongles, et les cheveux.

Elle peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant.

Ainsi, l'invention a pour objet l'utilisation d'une composition cosmétique de soin, de nettoyage, de maquillage ou déodorante comprenant au moins un hydroxystilbène.

Particulièrement l'invention a pour objet l'utilisation d'une composition cosmétique déodorante comprenant au moins un hydroxystilbène.

Encore plus particulièrement l'utilisation de la composition déodorante de l'invention est sous forme de stick.

De façon connue, la composition utilisée dans l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01% à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 % à 80 % en poids, et de préférence de 5 % à 50 % du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 % à 30 % en poids, et de préférence de 0,5 % à 20 % du poids total de la composition.

Comme huiles utilisables dans l'invention, on peut citer les huiles minérales, les huiles d'origine végétale (huile d'abricot, huile de tournesol), les huiles d'origine animale, les huiles de synthèse, les huiles siliconées (cyclométhicone) et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les hydratants tels que les polyols (par exemple la glycérine), les vitamines (par exemple le D-panthénol), les agents anti-inflammatoires, les agents apaisants (allantoïne, eau de bleuet), les filtres UVA et UVB, les agents matifiants (par exemple les polydiméthylorganosiloxanes partiellement réticulés vendus sous le nom KSG® par Shin Etsu), et leurs mélanges.

On peut aussi ajouter des actifs antirides et notamment des produits tenseurs tels que les protéines végétales et leurs hydrolysats, en particulier l'extrait de protéine de soja vendue sous le nom d'Eleseryl® par la société LSN ou le dérivé d'avoine vendu sous la dénomination Reductine® par la société Silab.

D'autres caractéristiques et avantages de l'invention ressortiront mieux des exemples qui suivent et des figures annexées, donnés à titre illustratif et non limitatif. Les proportions sont données en pourcentage pondéral.

### Exemple 1 : Mesure de l'activité du resvératrol sur l'adhésion de micro-organismes sur la peau ou sur les muqueuses humaines.

L'activité du resvératrol sur l'adhésion de micro-organismes sur la peau ou sur les muqueuses humaines est testée sur un modèle d'adhésion bactéries/peau, tel que décrit ci-après.

Ce modèle d'adhésion bactéries/peau *ex vivo* est basé sur l'utilisation d'explants de peau humaine intacte montée en sandwich entre une plaque 96 puits sans fond et un support en Plexiglas. Les puits individualisés étanches permettent de tester dans un format standard l'effet du resvératrol sur l'adhésion de *Staphylococcus epidermis* (bactéries résidentes) à la surface de la peau. Les bactéries sont radio marquées en phase de croissance par incorporation de 3H-thymidine. Cette flore bactérienne est ensuite déposée dans chaque puits. Après une heure d'incubation à 20°C, les puits sont lavés trois fois avec du tampon phosphate salin (PBS) ce qui permet d'éliminer les bactéries libres. Le resvératrol est ensuite ajouté à 0,1 µM et 10 µM et incubé 1 heure à 20°C. A l'issue de cette incubation le contenu de chaque puits est récupéré et compté en scintillation liquide (bactéries désorbées). Les puits sont ensuite lavés avec une solution chaotropique qui permet d'éluer les bactéries adhérentes. Les solutions de lavages sont également compté en scintillation liquide (bactéries adhérentes). Un témoin positif (Fucogel® Acide (poly-[(α-1,3)-Fuc-(α-1,3)-Gal-(α-1,3)-galacturonique]) est inclus à l'étude comme référence.

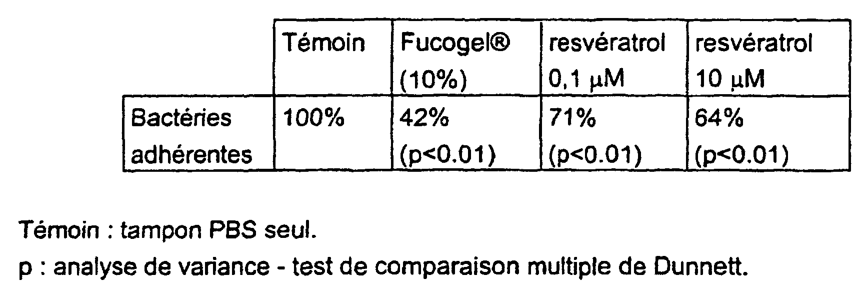

Le Fucogel® (contrôle positif) stimule la désorption des bactéries et inhibe l'adhésion. Le resvératrol à 0,1 µM et 10 µM inhibe l'adhésion des bactéries (29 et 36% d'inhibition respectivement).

En conclusion, les résultats présentés ci-dessus confirment bien le rôle du resvératrol dans la régulation de l'adhésion des bactéries sur la peau humaine. Cette activité est dose dépendante.

### Exemple 2 : Exemples de compositions selon l'invention. Ces compositions sont obtenues par les techniques habituelles couramment utilisées en cosmétique ou en pharmacie.

### Composition 1 : Lait pour le visage

- Huile de vaseline 7,00 g
- Resvératrol 0,50 g
- Monostéarate de glycéryle, stéarate de polyéthylène glycol (100 OE) 3,00 g
- Polymère carboxyvinylique 0,40 g
- Alcool stéarylique 0,70 g
- Protéines de soja 3,00 g
- NaOH 0,40 g
- Conservateur qs
- Eau qsp 100,00 g

Cette composition se présente sous forme d'un lait pour le visage, ayant de bonnes propriétés cosmétiques, et étant doux et confortable à utiliser. Le pH de la composition est d'environ 5,5.

### Composition 2 : Lotion antiseptique

- Resvératrol 3,00 g
- Palmitate d'éthyl-2 hexyle 10,00 g
- Cyclopentadiméthylsiloxane 20,00 g
- Butylène glycol 5,00 g
- Conservateur qs
- Eau qsp 100,00 g

Cette lotion, qui ne contient pas de tensioactif, est particulièrement bien adaptée au lavage de la peau.

### Composition 3 : Lait

- Palmitate d'octyle 35,00 g
- Glycérine 2,00 g
- Resvératrol 0,10 g
- Polymère réticulé acrylates C10-C30/alkylacrylates 0,10 g
- Triéthanolamine 0,10 g
- Acides aminés de blé 1,00 g
- Conservateur qs
- Eau qsp 100,00 g

Le lait obtenu, qui ne contient pas de tensioactif, possède de bonnes propriétés cosmétiques.

### Composition 4 Gel

- Glycérine 10,00 g
- Resvératrol 0,50 g
- Cocoamphodiacétate de disodium 1,00 g
- Conservateur qs
- Eau qsp 100,00 g

Le gel obtenu possède de bonnes propriétés cosmétiques.

### Composition 5 : Gel nettoyant à l'eau

- Butylène glycol 7,00 g
- Sarcosinate de lauroyl sodium 4,00 g
- Resvératrol 5,00g
- Triéthanolamine 0,80 g
- Carbomer 0,50 g
- Conservateur qs
- Eau qsp 100,00g

Le gel obtenu possède de bonnes propriétés cosmétiques.

### Composition 6 : Stick déodorant alcoolique

- Sodium stéarate 8,00 g
- Alcool éthylique 64,80 g
- Propylène glycol 10,00 g
- Myristate d'Isopropyle 5,00 g
- Resvératrol 2,00 g

### Composition 7 : Stick déodorant sans alcool

- Sodium stéarate 8,00 g
- Propylène glycol 10,00 g
- Coconut diéthanolamide 5,00 g
- PPG-3-Myristyl éther 65,00 g
- Resvératrol 5,00 g

## Revendications

1. Utilisation dans une composition cosmétique ou pour la préparation d'une composition dermatologique, en tant que principe actif, d'une quantité efficace d'au moins un hydroxystilbène, cet hydroxystilbène étant destiné à modifier l'adhésion des bactéries et/ou des levures sur la peau et/ou des muqueuses.

2. Utilisation selon la revendication 1, **caractérisée par le fait que** l'hydroxystilbène est destiné à prévenir, totalement ou partiellement, l'adhésion des bactéries et/ou des levures sur la peau et/ou des muqueuses.

3. Utilisation selon la revendication 1, **caractérisée par le fait que** l'hydroxystilbène est destiné à faciliter le détachement des bactéries et/ou des levures de la peau et/ou des muqueuses.

4. Utilisation selon l'une quelconque des revendications précédentes, **caractérisée par le fait que** l'hydroxystilbène ou la composition est utilisé en application topique sur la peau et/ou les muqueuses.

5. Utilisation selon l'une quelconque des revendications précédentes, **caractérisée par le fait que** l'hydroxystilbène est destiné aux soins d'hygiène corporelle.

6. Utilisation selon l'une quelconque des revendications précédentes, **caractérisée par le fait que** l'hydroxystilbène ou la composition sont destinés aux soins d'hygiène intime.

7. Utilisation selon l'une quelconque des revendications précédentes, **caractérisée par le fait que** l'hydroxystilbène ou la composition sont destinés à diminuer les mauvaises odeurs.

8. Utilisation selon l'une quelconque des revendications précédentes, **caractérisée par le fait que** l'hydroxystilbène ou la composition sont destinés à lutter contre l'acné et/ou les pellicules.

9. Utilisation selon la revendication précédente, **caractérisée par le fait que** la composition est destinée à lutter contre l'acné juvénile.

10. Utilisation selon l'une quelconque des revendications précédentes, **caractérisée par le fait que** l'hydroxystilbène est présent en une quantité allant de 0,001 % à 10 % du poids total de la composition.

11. Utilisation selon la revendication précédente, **caractérisée par le fait que** l'hydroxystilbène est présent en une quantité allant de 0,05% à 5% du poids total de la composition.

12. Utilisation selon l'une quelconque des revendications précédentes, **caractérisée par le fait que** l'hydroxystilbène est choisi parmi :
le 4'-hydroxystilbène,
le 2',4'-dihydroxystilbène,
le 3',4'-dihydroxystilbène,
le 4,4'-dihydroxystilbène,
le 2',4',4-trihydroxystilbène,
le 3',4',4-trihydroxystilbène,
le 2,4,4'-trihydroxystilbène,
le 3,4,4'-trihydroxystilbène,
le 3,4',5-trihydroxystilbène,
le 2',3,4-trihydroxystilbène,
le 2,3',4-trihydroxystilbène,
le 2',2,4'-trihydroxystilbène,
le 2,4,4',5-tétrahydroxystilbène,
le 2',3,4',5-tétrahydroxystilbène,
le 2,2',4,4'-tétrahydroxystilbène,
le 3,3',4',5-tétrahydroxystilbène,
le 2,3',4,4'-tétrahydroxystilbène,
le 3,3',4,4'-tétrahydroxystilbène,
le 3,3',4',5, 5'-pentahydroxystilbène,
le 2,2',4,4',6-pentahydroxystilbène,
le 2,3',4,4',6-pentahydroxystilbène,
le 2,2',4,4',6,6'-hexahydroxystilbène.

13. Utilisation selon l'une quelconque des revendications précédentes, **caractérisée par le fait que** l'hydroxystilbène est du 3, 4', 5-trihydroxystilbène.

14. Utilisation selon l'une quelconque des revendications précédentes, **caractérisée par le fait que** la composition se présente sous forme d'une lotion, d'un gel aqueux, d'un sérum, d'une émulsion ou d'une dispersion de vésicules lipidiques.

15. Utilisation selon l'une quelconque des revendications précédentes, **caractérisée par le fait que** la composition contient, en outre, au moins un adjuvant choisi parmi les gélifiants, les actifs, les conservateurs, les antioxydants, les solvants, les parfums, les charges, les filtres, les pigments, les agents chélateurs, les absorbeurs d'odeur, les matières colorantes, les agents matifiants et leurs mélanges.

16. Procédé de traitement cosmétique pour traiter les désordres liés à l'adhésion de bactéries et/ou des levures consistant à appliquer sur la peau une composition cosmétique comprenant au moins un hydroxystilbène dans un milieu cosmétiquement acceptable.

17. Stick déodorant comprenant au moins un hydroxystilbène tel que défini dans les revendications 1 à 13.

## Patentansprüche

1. Verwendung mindestens eines Hydroxystilben in einer wirksamen Menge als Wirkstoff in einer dermatologischen Zusammensetzung oder für die Herstellung einer dermatologischen Zusammensetzung, wobei das Hydroxystilben dazu vorgesehen ist, die Adhäsion von Bakterien und/oder Hefen auf der Haut und/oder den Schleimhäuten zu verändern.

2. Verwendung nach Anspruch 1, **dadurch gekennzeichnet, dass** das Hydroxystilben dazu dienen soll, die Adhäsion von Bakterien und/oder Hefen auf der Haut und/oder den Schleimhäuten ganz oder teilweise zu verhindern.

3. Verwendung nach Anspruch 1, **dadurch gekennzeichnet, dass** das Hydroxystilben dafür vorgesehen ist, das Ablösen von Bakterien und/ oder Hefen von der Haut und/ oder den Schleimhäuten zu erleichtern.

4. Verwendung nach einem der vorhergehenden Ansprüche, **dadurch gekennzeichnet, dass** das Hydroxystilben oder die Zusammensetzung topisch auf die Haut und/ oder die Schleimhäute aufgebracht werden.

5. Verwendung nach einem der vorhergehenden Ansprüche, **dadurch gekennzeichnet, dass** das Hydroxystilben für die Körperpflege vorgesehen ist.

6. Verwendung nach einem der vorhergehenden Ansprüche, **dadurch gekennzeichnet, dass** das Hydroxystilben oder die Zusammensetzung für die Intimpflege vorgesehen sind.

7. Verwendung nach einem der vorhergehenden Ansprüche, **dadurch gekennzeichnet, dass** das Hydroxystilben oder die Zusammensetzung für die Abschwächung unangenehmer Gerüche vorgesehen sind.

8. Verwendung nach einem der vorhergehenden Ansprüche, **dadurch gekennzeichnet, dass** das Hydroxystilben oder die Zusammensetzung für die Bekämpfung von Akne und/oder Schuppen vorgesehen sind.

9. Verwendung nach dem vorhergehenden Anspruch, **dadurch gekennzeichnet, dass** die Zusammensetzung für die Bekämpfung von Akne juvenilis dienen soll.

10. Verwendung nach einem der vorhergehenden Ansprüche, **dadurch gekennzeichnet, dass** das Hydroxystilben in einer Menge von 0,001 bis 10 % des Gesamtgewichts der Zusammensetzung enthalten ist.

11. Verwendung nach dem vorhergehenden Anspruch, **dadurch gekennzeichnet, dass** das Hydroxystilben in einer Menge von 0,05 bis 5 % des Gesamtgewichts der Zusammensetzung verwendet wird.

12. Verwendung nach einem der vorhergehenden Ansprüche, **dadurch gekennzeichnet, dass** das Hydroxystilben unter den folgenden Verbindungen ausgewählt ist:
4'-Hydroxystilben,
2',4'-Dihydroxystilben,
3',4'-Dihydroxystilben,
4,4'-Dihydroxystilben,
2',4',4-Trihydroxystilben,
3',4',4-Trihydroxystilben,
2,4,4'-Trihydroxystilben,
3,4,4'-Trihydroxystilben,
3,4',5-Trihydroxystilben,
2',3,4-Trihydroxystilben,
2,3',4-Trihydroxystilben,
2',2,4'-Trihydroxystilben,
2,4,4',5-Tetrahydroxystilben,
2',3,4',5-Tetrahydroxystilben,
2,2',4,4'-Tetrahydroxystilben,
3,3',4',5-Tetrahydroxystilben,
2,3',4,4'-Tetrahydroxystilben,
3,3',4,4'-Tetrahydroxystilben,
3,3',4',5,5'-Pentahydroxystilben,
2,2',4,4',6-Pentahydroxystilben,
2,3',4,4',6-Pentahydroxystilben,
2,2',4,4',6,6'-Hexahydroxystilben.

13. Verwendung nach einem der vorhergehenden Ansprüche, **dadurch gekennzeichnet, dass** es sich bei dem Hydroxystilben um das 3,4',5-Trihydroxystilben handelt.

14. Verwendung nach einem der vorhergehenden Ansprüche, **dadurch gekennzeichnet, dass** die Zusammensetzung in Form einer Lotion, eines wässrigen Gels, eines Serums, einer Emulsion oder einer Dispersion von Lipidvesikeln vorliegt.

15. Verwendung nach einem der vorhergehenden Ansprüche, **dadurch gekennzeichnet, dass** die Zusammensetzung außerdem mindestens einen Zusatzstoff enthält, der unter den Gelbildnern, Wirkstoffen, Konservierungsmitteln, Antioxidantien, Lösungsmitteln, Parfums, Füllstoffen, Filtern, Pigmenten, Chelatbildnern, Geruchsabsorbern, Farbmitteln, Mattierungsmitteln und den Gemischen dieser Zusatzstoffe ausgewählt ist.

16. Verfahren zur kosmetischen Behandlung für die Behandlung von Störungen, die mit der Adhäsion von Bakterien und/oder Hefen zusammenhängen, das darin besteht, auf die Haut eine kosmetische Zusammensetzung aufzutragen, die in einem kosmetisch akzeptablen Medium mindestens ein Hydroxystilben enthält.

17. Deodorant in Form eines Stifts mit mindestens einem Hydroxystilben nach einem der Ansprüche 1 bis 13.

## Claims

1. Use, in a cosmetic composition or for the preparation of a dermatological composition, as active ingredient, of an effective quantity of at least one hydroxystilbene, this hydroxystilbene being intended to modify the adhesion of bacteria and/or yeasts to the skin and/or mucous membranes.

2. Use according to Claim 1, **characterized in that** the hydroxystilbene is intended to prevent, completely or partially, the adhesion of bacteria and/or yeasts to the skin and/or mucous membranes.

3. Use according to Claim 1, **characterized in that** the hydroxystilbene is intended to facilitate the detachment of bacteria and/or yeasts from the skin and/or mucous membranes.

4. Use according to any one of the preceding claims, **characterized in that** the hydroxystilbene or the composition is used as a topical application to the skin and/or mucous membranes.

5. Use according to any one of the preceding claims, **characterized in that** the hydroxystilbene is intended for body hygiene treatments.

6. Use according to any one of the preceding claims, **characterized in that** the hydroxystilbene or the composition are intended for intimate hygiene treatments.

7. Use according to any one of the preceding claims, **characterized in that** the hydroxystilbene or the composition are intended to reduce bad odours.

8. Use according to any one of the preceding claims, **characterized in that** the hydroxystilbene or the composition are intended to combat acne and/or dandruff.

9. Use according to the preceding claim, **characterized in that** the composition is intended to combat juvenile acne.

10. Use according to any one of the preceding claims, **characterized in that** the hydroxystilbene is present in a quantity ranging from 0.001% to 10% of the total weight of the composition.

11. Use according to the preceding claim, **characterized in that** the hydroxystilbene is present in a quantity ranging from 0.05% to 5% of the total weight of the composition.

12. Use according to any one of the preceding claims, **characterized in that** the hydroxystilbene is chosen from:
4'-hydroxystilbene,
2',4'-dihydroxystilbene,
3',4'-dihydroxystilbene,
4,4'-dihydroxystilbene,
2',4',4-trihydroxystilbene,
3',4',4-trihydroxystilbene,
2,4,4'-trihydroxystilbene,
3,4,4'-trihydroxystilbene,
3,4',5-trihydroxystilbene,
2',3,4-trihydroxystilbene,
2,3',4-trihydroxystilbene,
2',2,4'-trihydroxystilbene,
2,4,4',5-tetrahydroxystilbene,
2',3,4',5-tetrahydroxystilbene,
2,2',4,4'-tetrahydroxystilbene,
3,3',4',5-tetrahydroxystilbene,
2,3',4,4'-tetrahydroxystilbene,
3,3',4,4'-tetrahydroxystilbene,
3,3',4',5,5'-pentahydroxystilbene,
2,2',4,4',6-pentahydroxystilbene,
2,3',4,4',6-pentahydroxystilbene,
2,2',4,4',6,6'-hexahydroxystilbene.

13. Use according to any one of the preceding claims, **characterized in that** the hydroxystilbene is 3,4',5-trihydroxystilbene.

14. Use according to any one of the preceding claims, **characterized in that** the composition is provided in the form of a lotion, an aqueous gel, a serum, an emulsion or a dispersion of lipid vesicles.

15. Use according to any one of the preceding claims, **characterized in that** the composition contains, in addition, at least one adjuvant chosen from gelling agents, active agents, preservatives, antioxidants, solvents, perfumes, fillers, screening agents, pigments, chelating agents, odour absorbers, colouring matter, matting agents and mixtures thereof.

16. Method of cosmetic treatment for treating disorders linked to the adhesion of bacteria and/or yeasts consisting in applying to the skin a cosmetic composition comprising at least one hydroxystilbene in a cosmetically acceptable medium.

17. Deodorant stick comprising at least one hydroxystilbene as defined in Claims 1 to 13.
